# FASCICULE DE BREVET EUROPEEN

(11) **EP 1 029 060 B1**
(45) Date de publication et mention de la délivrance du brevet: **29.04.2009**
(21) Numéro de dépôt: 98954544.7
(22) Date de dépôt: 09.11.1998
(51) Int. Cl.: C12N 15/53, C12N 15/62, C12N 15/82, C12N 5/10, C12N 9/02, A01H 5/00, A01H 5/10

(54) **HYDROXY-PHENYL PYRUVATE DIOXYGENASE CHIMERE, SEQUENCE D'ADN ET OBTENTION DE PLANTES CONTENANT UN TEL GENE, TOLERANTES AUX HERBICIDES**
CHIMÄRE HYDROXYPHENYLPYRUVATDIOXYGENASE, DNA SEQUENZ UND HERBIZIDTOLERANTE PFLANZEN, DIE EIN SOLCHES GEN BESITZEN
CHIMERIC HYDROXY-PHENYL PYRUVATE DIOXYGENASE, DNA SEQUENCE AND METHOD FOR OBTAINING PLANTS CONTAINING SUCH A GENE, WITH HERBICIDE TOLERANCE

(30) Priorité: 07.11.1997 FR 9714264
(43) Date de publication de la demande: 23.08.2000
(73) Titulaire: Bayer CropScience S.A., 69009 Lyon (FR)
(72) Inventeur: BOUDEC, Philippe, F-69009 Lyon (FR); BOURDON, Hélène, F-69130 Ecully (FR); DUMAS, Florence, F-69250 Fleurieu sur Saône (FR); RODGERS, Matthew, Ongar, Essex CM5 0HW (GB); SAILLAND, Alain, F-69009 Lyon (FR)
(74) Mandataire: Guitton, Carole
(86) Numéro de dépôt international: PCT/FR1998/002391
(87) Numéro de publication internationale: WO 1999/024586

(56) Documents cités:
- EP-A- 0 252 666
- WO-A-96/32484
- WO-A-96/38567
- WO-A-97/27285
- LEE M. ET AL.: "The C-terminal of rat 4-hydroxyphenylpyruvate dioxygenase is indispensable for enzyme activity" FEBS LETTERS, vol. 393, no. 2,3, 16 septembre 1996, pages 269-272, XP002070559
- GARCIA I. ET AL.: "Subcellular localization and purification of a p-hydroxyphenylpyruvate dioxygenase from cultured carrot cells and characterization of the corresponding cDNA" BIOCHEMICAL JOURNAL, (1997 AUG 1) 325 ( PT 3) 761-9. JOURNAL CODE: 9YO. ISSN: 0264-6021., XP002070560

## Description

La présente invention concerne une séquence d'acide nucléique codant pour une hydroxy-phényl pyruvate dioxygénase (HPPD) chimère, un gène chimère contenant cette séquence comme séquence codante et son utilisation pour l'obtention de plantes résistantes à certains herbicides.

Les hydroxy-phényl pyruvate dioxygénases sont des enzymes qui catalysent la réaction de transformation du para-hydroxy-phényl-pyruvate (HPP) en homogentisate. Cette réaction a lieu en présence de fer (Fe²⁺) en présence d'oxygène (Crouch N.P. & al., Tetrahedron, 53, 20, 6993-7010, 1997). On peut émettre l'hypothèse que les HPPD contiennent un site actif apte à catalyser cette réaction dans lequel viennent se lier le fer, le substrat et la molécule d'eau, un tel site actif n'ayant jamais été décrit à ce jour.

On connaît par ailleurs certaines molécules inhibitrices de cette enzyme, qui viennent se fixer à l'enzyme de manière compétitive pour inhiber la transformation de l'HPP en homogentisate. Certaines de ces molécules ont trouvé un emploi comme herbicides, dans la mesure où l'inhibition de la réaction dans les plantes conduit à un blanchiment des feuilles des plantes traitées, et à la mort des dites plantes (Pallett K. E. et al. 1997 Pestic. Sci. 50 83-84). De tels herbicides ayant pour cible l'HPPD décrits dans l'état de la technique sont notamment les isoxazoles (EP 418 175, EP 470 856, EP 487 352, EP 527 036, EP 560 482, EP 682 659, US 5 424 276) en particulier l'isoxaflutole, herbicide sélectif du maïs, les dicétonitriles (EP 496 630, EP 496 631), en particulier la 2-cyano-3-cyclopropyl-1-(2-SO₂ CH₃-4-CF₃ phényl) propane-1,3-dione et la 2-cyano-3-cyclopropyl-1-(2-SO₂ CH₃-4-2,3 Cl₂ phényl) propane-1, 3-dione, les tricétones (EP 625 505, EP 625 508, US 5,506,195), en particulier la sulcotrione ou encore les pyrazolinates.

Pour rendre les plantes tolérantes aux herbicides, on dispose de trois stratégies principales, (1) la détoxification de l'herbicide par une enzyme venant transformer l'herbicide, ou son métabolite actif, en produits de dégradation non toxique, comme par exemple les enzymes de tolérance au bromoxynil ou au basta (EP 242 236, EP 337 899) ; (2) la mutation de l'enzyme cible en une enzyme fonctionnelle moins sensible à l'herbicide, ou son métabolite actif, comme par exemple les enzymes de tolérance au glyphosate (EP 293 356, Padgette S. R. & al., J. Biol. Chem., 266, 33, 1991) ; ou (3) la surexpression de l'enzyme sensible, de manière à produire dans la plante des quantités suffisantes d'enzyme cible au regard des constantes cinétiques de cette enzyme vis à vis de l'herbicide de manière à avoir suffisamment d'enzyme fonctionnelle, malgré la présence de son inhibiteur.

C'est cette troisième stratégie qui a été décrite pour obtenir avec succès des plantes tolérantes aux inhibiteurs d'HPPD (WO 96/38567), étant entendu que pour la première fois une stratégie de simple surexpression de l'enzyme cible sensible (non mutée) était employée avec succès pour conférer aux plantes une tolérance à un niveau agronomique à un herbicide.

Malgré le succès obtenu avec cette stratégie de simple surexpression de l'enzyme cible, il reste nécessaire de diversifier le système de tolérance aux inhibiteurs d'HPPD, pour obtenir une tolérance quelques soient les conditions de culture des plantes tolérantes ou les doses commerciales d'application des herbicides dans les champs. On connaît de l'état de la technique (WO 96/38567) que des enzymes d'origine différente (plantes, bactéries, champignons) ont des séquences protéiques primaires substantiellement différentes, alors que ces enzymes ont une fonction identique et des caractéristiques cinétiques essentiellement similaires ou voisines.

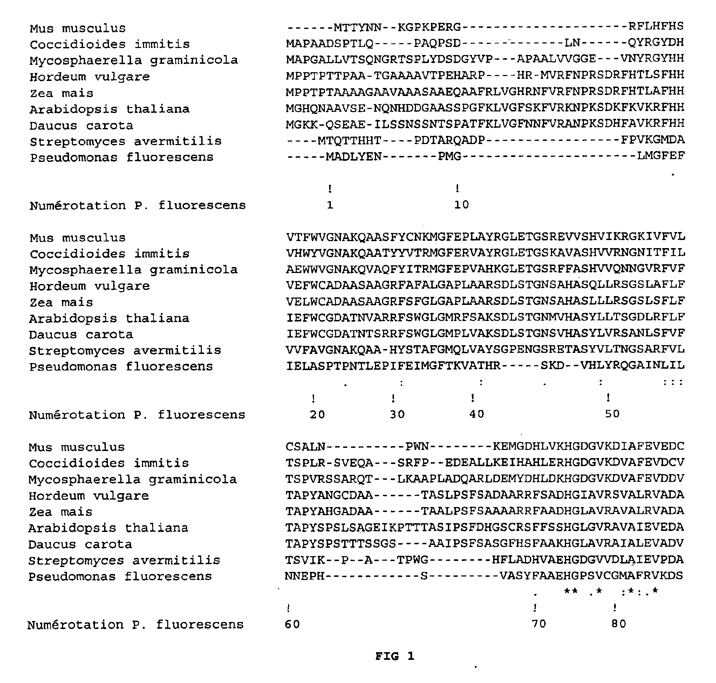

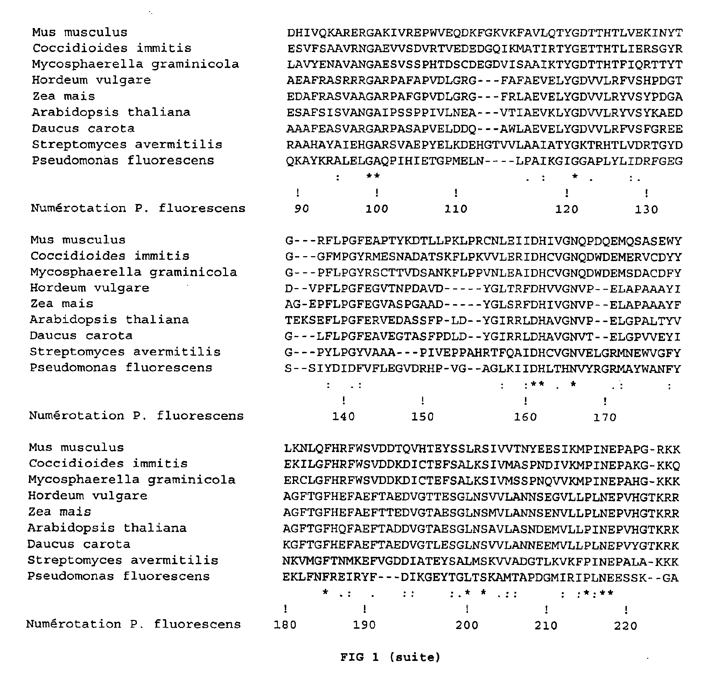

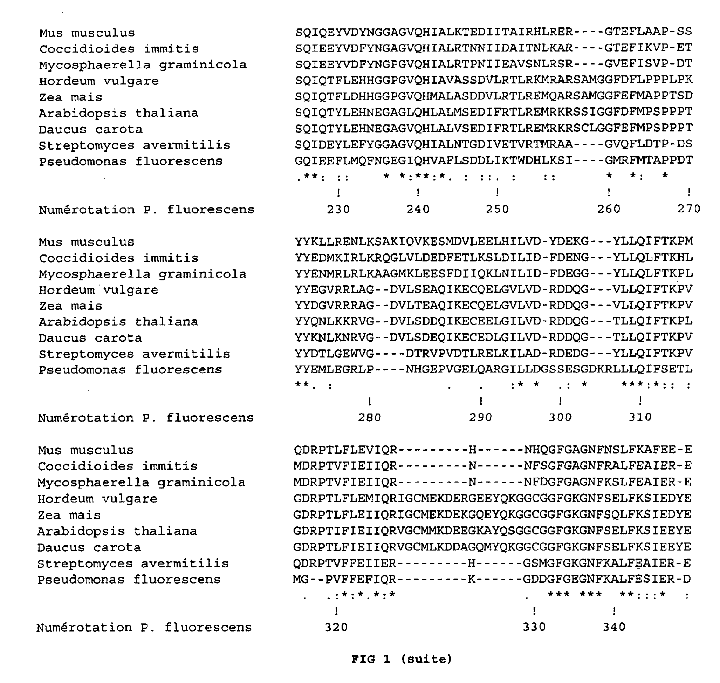

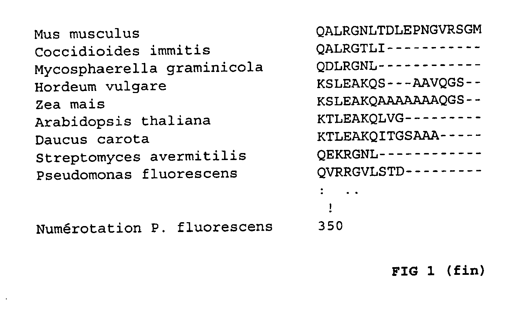

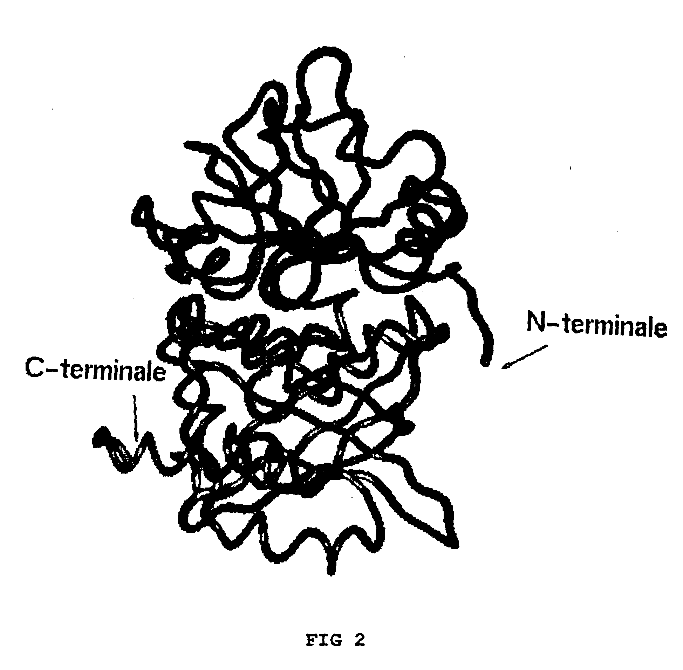

On a maintenant constaté que toutes ces HPPD ont d'une part un grand nombre d'homologies de séquence dans leur partie C-terminale (figure 1), et d'autre part une structure tertiaire (tridimensionnelle) essentiellement similaire (figure 2). Au regard du caractère compétitif de l'inhibition, on émet l'hypothèse que les inhibiteurs d'HPPD viennent se lier à l'enzyme dans le site actif de cette dernière, ou à proximité de celui-ci, de manière à bloquer l'accès de l'HPP à ce site actif et empêcher sa transformation en présence de fer et d'eau. On a maintenant constaté qu'en effectuant une mutation de l'enzyme dans sa partie C-terminale, il était possible d'obtenir des HPPD fonctionnelles moins sensibles aux inhibiteurs d'HPPD, de manière que leur expression dans les plantes permette une tolérance améliorée aux inhibiteurs d'HPPD. Au regard de ces éléments, ont peut donc conclure que le site actif de l'enzyme est localisé dans sa partie C-terminale, sa partie N-terminale assurant essentiellement sa stabilité et son oligomérisation (l'HPPD de *Pseudomonas* est un tétramère, celles de plantes sont des dimères).

On a maintenant constaté qu'il était possible de réaliser une enzyme chimère en associant la partie N-terminale d'une première enzyme la partie C-terminale d'une seconde enzyme, pour obtenir une nouvelle HPPD chimère fonctionnelle, permettant de choisir chaque partie pour des propriétés particulières, comme par exemple choisir la partie N-terminale d'une première enzyme pour ses propriétés de stabilité dans une cellule donnée (végétale, bactérienne, etc.) et la partie C-terminale d'une deuxième enzyme pour ses propriétés cinétiques (activité, tolérance aux inhibiteurs, etc.).

La présente invention concerne donc en premier lieu une HPPD chimère, qui tout en étant fonctionnelle, c'est à dire conservant ses propriétés de catalyse de la transformation de l'HPP en homogentisate, comprend la partie N-terminale d'une première HPPD associée à la partie C-terminale d'une deuxième HPPD.

Selon un mode préférentiel de réalisation de l'invention, la partie N-terminale de l'HPPD chimère provient d'une HPPD de plante, ladite plante étant préférentiellement choisie parmi les plantes dicotylédones, notamment *Arabidopsis thaliana* ou *Daucus carotta*, ou encore de monocotylédones comme le mais ou le blé.

Selon un autre mode préférentiel de réalisation de l'invention, la partie C-terminale de l'HPPD chimère provient d'une HPPD de plante, telle que définie ci-dessus, ou d'une HPPD de micro-organisme, notamment une bactéie, en particulier *Pseudomonas,* plus particulièrement *Pseudomonas fluorescens*, ou de champignon, cette partie C-terminale pouvant être naturelle ou mutée par la substitution de un ou plusieurs acides aminés de la partie C-terminale de l'HPPD d'origine, notamment pour la rendre moins sensible aux inhibiteurs d'HPPD.

Plusieurs HPPD et leur séquence primaire ont été décrites dans l'état de la technique, notamment les HPPD de bactéries comme *Pseudomonas* (Rüetschi & al., Eur. J. Biochem., 205, 459-466, 1992, WO 96/38567), de plantes comme d'*Arabidopsis* (WO 96/38567, Genebank AF047834) ou de carotte (WO 96/38567, Genebank 87257), de *Coccicoides* (Genebank COITRP), ou de mammifères comme la souris ou le cochon.

L'alignement de ces séquences connues, par les moyens usuels de la technique, comme par exemple la méthode décrite par Thompson, J.D. & al. (CLUSTAL W: improving the sensitivity of progressive multiple sequence alignment through sequence weighting, positions-specific gap penalties and weight matrix choice. Nucleic Acids Research, 22:4673-4680, 1994), et l'accès à ces programmes informatique d'alignement de séquence accessibles par exemple via internet, l'homme du métier peut définir les homologies de séquence par rapport à une séquence de référence, et retrouver les acides aminés clés, ou encore définir des régions communes, notamment permettant de définir une région C-terminale et une région N-terminale à partir de cette séquence de référence.

Pour la présente invention, la séquence de référence est la séquence de *Pseudomonas,* toutes les définitions et indications de positions d'acides aminés particuliers étant faites par rapport à la séquence primaire d'HPPD de *Pseudomonas.* La figure 1 en annexe représente un alignement de plusieurs séquences d'HPPD décrites dans l'état de la technique, alignées par rapport à la séquence d'HPPD de *Pseudomonas* comme référence, comprenant les séquence d'HPPD de *Streptomyces avermitilis* (Genebank SAV11864), de *Daucus carota* (Genebank DCU 87257), d'*Arabidopsis thaliana* (Genebank AF047834), de *Zea mais,* de *Hordeum vulgare* (Genebank HVAJ693), de *Mycosphaerella graminicola* (Genebank AF038152), de *Coccicoides immitis* (Genebank COITRP) et de *Mus musculus* (Genebank MU54HD). La numérotation des acides aminés de la séquence de *Pseudomonas* est donnée sur cette figure, de même que les acides aminés communs à ces séquences, désignés par une astérisque. Sur la base d'un tel alignement il est aisé d'identifier à partir de la définition de l'acide aminé de *Pseudomonas* par sa position et sa nature, la position de l'acide aminé correspondant dans une autre séquence d'HPPD (l'alignement de séquences de différentes origines, plantes, mammifères, bactéries, montrant que cette méthode d'alignement bien connue de l'homme du métier peut être généralisée à toute autre séquence). Un alignement de différentes séquences d'HPPD est également décrit dans la demande de brevet WO 97/49816.

La partie C-terminale des HPPD, siège du site actif de l'enzyme, se distingue par un peptide de liaison de sa partie N-terminale comme le montre la représentation schématique de la structure ternaire du monomère de l'HPPD de *Pseudomonas* représentée en figure 2. Cette structure a été obtenue par les méthodes usuelles d'étude de la diffraction aux rayons X de cristaux Le peptide de liaison va permettre de définir l'extrémité N-terminale de la partie C-terminale de l'enzyme, ledit peptide se situant entre les acides aminés 145 et 157 pour *Pseudomonas* (c.f. figure 1).

La partie C-terminale peut donc être définie comme constituée par la séquence définie d'une part par le peptide de liaison, et d'autre part par l'extrémité C-terminale de l'enzyme. Sur l'alignement de séquences représenté sur la figure 1 en annexe, on remarque pour toutes les séquences deux acides aminés en position 161 et 162 pour la séquence de *Pseudomonas,* (D = Asp161 et H = His162). Par référence à l'HPPD de *Pseudomonas,* on peut donc définir que le peptide de liaison représentant l'extrémité N-terminale de la partie C-terminale de l'HPPD se situe entre environ 5 et 15 acides aminés en amont de l'acide aminé Asp161.

La présente invention concerne également une séquence d'acide nucléique codant pour une HPPD chimère décrite ci-dessus. Selon la présente invention, on entend par " séquence d'acide nucléique " une séquence nucléotidique pouvant être de type ADN ou ARN, de préférence de type ADN, notamment double brin, qu'elle soit d'origine naturelle ou synthétique, notamment une séquence d'ADN pour laquelle les codons codant pour l'HPPD chimère selon l'invention auront été optimisés en fonction de l'organisme hôte dans lequel elle sera exprimée, ces méthodes d'optimisations étant bien connues de l'homme du métier.

Les séquences codant pour chaque HPPD d'origine de l'HPPD chimère selon l'invention, sont d'origine végétale, notamment issu de plantes dicotyledones telles que tabac, *Arabidopsis*, d'ombellifères telles que *Daucus carotta* ou encore monocotyledones telles que le *Zea maïs* ou le blé. Les séquences codantes et le moyen de les isoler et cloner sont décrits dans les références citées auparavant, dont le contenu est incorporé ici par référence.

Les séquences codantes des parties N-terminales et C-terminales de l'HPPD chimère selon l'invention peuvent être assemblées par toute méthode usuelle de construction et d'assemblage de fragments d'acides nucléiques bien connues de l'homme du métier et largement décrites dans la littérature, illustrées notamment par les exemples de réalisation de l'invention.

La présente invention concerne donc également un procédé de préparation d'une séquence d'acide nucléique codant pour une HPPD chimère selon l'invention, ledit procédé étant défini ci-dessus.

L'invention a encore pour objet l'utilisation d'une séquence d'acide nucléique codant pour une HPPD chimère selon l'invention dans un procédé pour la transformation des plantes, comme gène marqueur ou comme séquence codante permettant de conférer à la plante une tolérance aux herbicides inhibiteurs d'HPPD. Cette séquence peut bien entendu également être utilisée en association avec d'autre(s) gène(s) marqueur(s) et/ou séquence(s) codante(s) pour une ou plusieurs propriétés agronomiques.

La présente invention concerne également un gène chimère (ou cassette d'expression) comprenant une séquence codante ainsi que des éléments de régulation en position 5' et 3' hétérologues pouvant fonctionner dans un organisme hôte, en particulier les cellules végétales ou les plantes, la séquence codante comprenant au moins une séquence d'acide nucléique codant pour une HPPD chimère telle que définie précédemment.

Par organisme hôte, on entend tout organisme mono ou pluricellulaire, inférieur ou supérieur, dans lequel le gène chimère selon l'invention peut être introduit, pour la production d'HPPD chimère. Il s'agit en particulier de bactéries, par exemple *E. coli*, de levures, en particulier des genres *Saccharomyces* ou *Kluyveromyces, Pichia*, de champignons, en particulier *Aspergillus*, d'un baculovirus, ou de préférence des cellules végétales et des plantes.

Par "cellule végétale", on entend selon l'invention toute cellule issue d'une plante et pouvant constituer des tissus indifférenciés tels que des cals, des tissus différenciés tels que des embryons, des parties de plantes, des plantes ou des semences.

On entend par "plante" selon l'invention, tout organisme multicellulaire différencié capable de photosynthèse, en particulier monocotylédones ou dicotylédones, plus particulièrement des plantes de culture destinées ou non à l'alimentation animale ou humaine, comme le maïs, le blé, le colza, le soja, le riz, la canne à sucre, la betterave, le tabac, le coton, etc.

Les éléments de régulation nécessaires à l'expression de la séquence d'acide nucléique codant pour une HPPD sont bien connus de l'homme du métier en fonction de l'organisme hôte. Ils comprennent notamment des séquences promotrices, des activateurs de transcription, des séquences terminatrices, y compris des codons start et stop. Les moyens et méthodes pour identifier et sélectionner les éléments de régulation sont bien connus de l'homme du métier et largement décrits dans la littérature.

L'invention concerne plus particulièrement la transformation des plantes. Comme séquence de régulation promotrice dans les plantes, on peut utiliser toute séquence promotrice d'un gène s'exprimant naturellement dans les plantes en particulier un promoteur s'exprimant notamment dans les feuilles des plantes, comme par exemple des promoteurs dits constitutifs d'origine bactérienne, virale ou végétale tel que, par exemple un promoteur d'histone tel que décrit dans la demande EP 0 507 698, ou un promoteur d'actine de riz., d'un gène de virus de plante tel que, par exemple, celui de la mosaïque du choux fleur (CAMV 19S ou 35S), ou encore des promoteurs dits lumière dépendants comme celui d'un gène de la petite sous-unité de ribulose-biscarboxylase/oxygénase (RuBisCO) de plante, ou tout promoteur convenable connu pouvant être utilisé.

Selon l'invention, on peut également utiliser, en association avec la séquence de régulation promotrice, d'autres séquences de régulation, qui sont situées entre le promoteur et la séquence codante, telles que des activateurs de transcription ("enhancer"), comme par exemple l'activateur de translation du virus de la mosaïque du tabac (TMV) décrit dans la demande WO 87/07644, ou du virus etch du tabac (TEV) décrit par Carrington & Freed.

Comme séquence de régulation terminatrice ou de polyadénylation, on peut utiliser toute séquence correspondante d'origine bactérienne, comme par exemple le terminateur nos d'*Agrobacterium tumefaciens*, ou encore d'origine végétale, comme par exemple un terminateur d'histone tel que décrit dans la demande EP 0 633 317.

Selon un mode particulier de réalisation de l'invention, on emploie en 5' de la séquence d'acide nucléique codant pour une HPPD chimère, une séquence d'acide nucléique codant pour un peptide de transit, cette séquence étant disposée entre la région promotrice et la séquence codant pour l'HPPD chimère de manière à permettre l'expression d'une protéine de fusion peptide de transit/HPPD chimère, cette dernière étant définie précédemment. Le peptide de transit permet d'adresser l'HPPD chimère dans les plastes, plus particulièrement les chloroplastes, la protéine de fusion étant clivée entre le peptide de transit et l'HPPD chimère au passage de la membrane des plastes. Le peptide de transit peut être simple, comme un peptide de transit d'EPSPS (décrit dans le brevet US 5,188,642) ou un peptide de transit de celui de la petite sous-unité de ribulose-biscarboxylase/oxygénase ( ssu RuBisCO) d'une plante, éventuellement comprenant quelques acides aminés de la partie N-terminale de la ssu RuBisCO mature (EP 189 707) ou encore un peptide de transit multiple comprenant un premier peptide de transit de plante fusionné à une partie de la séquence N-terminale d'une protéine mature à localisation plastidiale, fusionnée à un deuxième peptide de transit de plante tel que décrit dans le brevet EP 508 909, et plus particulièrement le peptide de transit optimisé comprenant un peptide de transit de la ssu RuBisCO de tournesol fusionné à 22 acides aminés de l'extrémité N-terminale de la ssu RuBisCO de maïs fusionnée au peptide de transit de la ssu RuBisCO de maïs tel que décrit avec sa séquence codante dans le brevet EP 508 909.

La présente invention concerne également la protéine de fusion peptide de transit/HPPD chimère, les deux éléments de cette protéine de fusion étant définis plus haut.

La présente invention concerne également un vecteur de clonage et/ou d'expression pour la transformation d'un organisme hôte contenant au moins un gène chimère tel que défini ci-dessus. Ce vecteur comprend outre le gène chimère ci-dessus, au moins une origine de réplication. Ce vecteur peut être constitué par un plasmide, un cosmide, un bactériophage ou un virus, transformés par l'introduction du gène chimère selon l'invention. De tels vecteurs de transformation en fonction de l'organisme hôte à transformer sont bien connus de l'homme du métier et largement décrits dans la littérature. Pour la transformation des cellules végétales ou des plantes, il s'agira notamment d'un virus qui peut être employé pour la transformation des plantes développées et contenant en outre ses propres éléments de réplication et d'expression. De manière préférentielle, le vecteur de transformation des cellules végétales ou des plantes selon l'invention est un plasmide.

L'invention a encore pour objet un procédé de transformation des organismes hôtes, en particulier des cellules végétales par intégration d'au moins une séquence d'acide nucléique ou un gène chimère tels que définis ci-dessus, transformation qui peut être obtenue par tout moyen connu approprié, amplement décrit dans la littérature spécialisée et notamment les références citées dans la présente demande, plus particulièrement par le vecteur selon l'invention.

Une série de méthodes consiste à bombarder des cellules, des protoplastes ou des tissus avec des particules auxquelles sont accrochées les séquences d'ADN. Une autre série de méthodes consiste à utiliser comme moyen de transfert dans la plante un gène chimère inséré dans un plasmide Ti d'*Agrobacterium tumefaciens* ou Ri *d'Agrobacterium rhizogenes*. D'autres méthodes peuvent être utilisées telles que la micro-injection ou l'électroporation, ou encore la précipitation directe au moyen de PEG. L'homme du métier fera le choix de la méthode appropriée en fonction de la nature de l'organisme hôte, en particulier de la cellule végétale ou de la plante.

La présente invention a encore pour objet les organismes hôtes, en particulier cellules végétales ou plantes, transformés et contenant un gène chimère comprenant une séquence codante pour une HPPD chimère définie ci-dessus.

La présente invention a encore pour objet les plantes contenant des cellules transformées, en particulier les plantes régénérées à partir des cellules transformées. La régénération est obtenue par tout procédé approprié qui dépende de la nature de l'espèce, comme par exemple décrit dans les références ci-dessus. Pour les procédés de transformation des cellules végétales et de régénération des plantes, on citera notamment les brevets et demandes de brevet suivants: US 4,459,355, US 4,536,475, US 5,464,763, US 5,177,010, US 5,187,073, EP 267,159, EP 604 662, EP 672 752, US 4,945,050, US 5,036,006, US 5,100,792, US 5,371,014, US 5,478,744, US 5,179,022, US 5,565,346, US 5,484,956, US 5,508,468, US 5,538,877, US 5,554,798, US 5,489,520, US 5,510,318, US 5,204,253, US 5,405,765, EP 442 174, EP 486 233, EP 486 234, EP 539 563, EP 674 725, WO 91/02071 et WO 95/06128.

La présente invention concerne également les plantes transformées issues de la culture et/ou du croisement des plantes régénérées ci-dessus, ainsi que les graines de plantes transformées.

Les plantes transformées pouvant être obtenues selon l'invention peuvent être du type monocotylédones telles que par exemple les céréales, la canne à sucre, le riz et le maïs ou dicotyledones comme par exemple le tabac, la soja, le colza, le coton, la betterave, le trèfle, etc.

L'invention a aussi pour objet un procédé de désherbage sélectif de plantes, notamment de cultures, à l'aide d'un inhibiteur de l'HPPD notamment un herbicide définit auparavant, **caractérisé en ce qu**'on applique cet herbicide sur des plantes transformées selon l'invention, tant en présemis, en prélevée qu'en postlevée de la culture.

La présente invention concerne également un procédé de contrôle des mauvaises herbes dans une surface d'un champ comprenant des graines ou des plantes transformées avec le gène chimère selon l'invention, lequel procédé consiste à appliquer dans la dite surface du champ une dose toxique pour les dites mauvaises herbes d'un herbicide inhibiteur d'HPPD, sans toutefois affecter de manière substantielle les graines ou plantes transformée avec le dit gène chimère selon l'invention.

La présente invention concerne également un procédé de culture des plantes transformées selon l'invention avec un gène chimère selon l'invention lequel procédé consiste à planter les graines des dites plantes transformées dans une surface d'un champ approprié pour la culture des dites plantes, à appliquer sur la dite surface du dit champ une dose toxique pour les mauvaises herbes d'un herbicide ayant pour cible l'HPPD défini ci-dessus en cas de présence de mauvaises herbes, sans affecter de manière substantielle les dites graines ou les dites plantes transformées, puis à récolter les plantes cultivées lorsqu'elles arrivent à la maturité souhaitée et éventuellement à séparer les graines des plantes récoltées.

Dans les deux procédés ci-dessus, l'application de l'herbicide ayant pour cible l'HPPD peut être faite selon l'invention, tant en présemis, en prélevée qu'en postlevée de la culture.

Par herbicide au sens de la présente invention on entend une matière active herbicide seule ou associée à un additif qui modifie son efficacité comme par exemple un agent augmentant l'activité (synergiste) ou limitant l'activité (en anglais safener). Les herbicides inhibiteurs d'HPPD sont en particulier définis auparavant. Bien entendu, pour leur application pratique, les herbicides ci-dessus sont associée de manière en soi connue aux adjuvants de formulations utilisés habituellement en agrochimie

Lorsque la plante transformée selon l'invention comprend un autre gène de tolérance à un autre herbicide (comme par exemple un gène codant pour une EPSPS chimère ou non conférant à la plante une tolérance au glyphosate), ou lorsque la plante transformée est naturellement insensible à un autre herbicides, le procédé selon l'invention peut comprendre l'application simultanée ou décalée dans le temps d'un inhibiteur d'HPPD en association avec ledit herbicide, par exemple le glyphosate.

L'invention a encore pour objet l'utilisation du gène chimère codant pour une HPPD chimère comme gène marqueur au cours du cycle "transformation-régénération" d'une espèce végétale et sélection sur l'herbicide ci-dessus

Les différents aspects de l'invention seront mieux compris à l'aide des exemples expérimentaux ci-dessous.

Toutes les méthodes ou opérations décrites ci-dessous dans ces exemples sont données à titre d'exemples et correspondent à un choix, effectué parmi les différentes méthodes disponibles pour parvenir au même résultat. Ce choix n'a aucune incidence sur la qualité du résultat et par conséquent, toute méthode adaptée peut être utilisée par l'homme de l'art pour parvenir au même résultat. La plupart des méthodes d'ingénierie des fragments d'ADN sont décrites dans "Current Protocols in Molecular Biology" Volumes 1 et 2, Ausubel F.M. et al , publiés par Greene Publishing Associates et Wiley -Interscience (1989) ou dans Molecular cloning, T.Maniatis, E.F.Fritsch, J.Sambrook,1982.

### Exemple 1 Test de criblage colorimétrique de mutants présentant une tolérance à la 2-cyano-3-cyclopropyl-1-(2-SO₂ CH₃-4-CF₃ phényl) propane-1,3-dione

pRP C : Le vecteur pRP A (décrit dans la demande WO 96/38567) contenant un fragment d'ADN génomique et la région codante du gène de l'HPPD de *Pseudomonas fluorescens* A32 a été digéré par Ncol, purifié puis ligué dans le vecteur d'expression pKK233-2 (Clontech) lui-même digéré par Ncol, site de clonage unique de ce vecteur. L'orientation du gène dans le vecteur pRP C ainsi obtenu, permettant l'expression sous le contrôle du promoteur *trc* à été vérifiée.

Un milieu de culture de type YT broth à 1% d'agarose (ultra pur Gibco BRL) et à 5mM de L-Tyrosine (Sigma), et contenant l'agent de sélection du vecteur pRP C ci-dessus est dispensé en plaque de 96 puits à raison de 100ul par puits. 10ul d'une culture de E.Coli en phase exponentielle de croissance contenant le vecteur pRP C est dispensée dans chaque puits. Après 16 heures à 37°C, les puits ne contenant que le milieu de culture ou ceux ensemencés avec une culture d'E.Coli contenant le vecteur PKK233-2 sont translucides, alors que les puits ensemencés avec une culture de E.Coli contenant le vecteur pRP C sont de couleur brune.

Une gamme a été réalisée avec milieu de culture identique contenant des concentrations variables (0mM à 14mM) du 2-cyano-3-cyclopropyl-1-(2-méthylsulfonyl-4-trifluorométhylphényl) propan-1,3-dione (EP 0 496 631) solubilisé dans l'eau et amené à pH7,5. Cette molécule est un dicétonitrile, reconnu comme étant un inhibiteur efficace d'activité HPPD (Pallett, K.E. et al 1997. Pestic. Sci. 50, 83-84 ). On observe une absence totale de coloration pour la culture bactérienne contenant le vecteur pRP C à 7mM du composé ci-dessus.

Des résultats identiques ont été obtenus en substituant, à la 2-cyano-3-cyclopropyl-1-(2-méthyl-4-trifluorométhylphényl)propan-1,3-dione, la 2-cyano-3-cyclopropyl-1-(2-méthylsulfonyl -4-(méthylthio) phényl) propan-1,3-dione, et la 2-(2-chloro-3-ethoxy-4-(éthylsulfonyl) benzoyl)-5-méthyl-1,3-cyclohexadione,(WO 93/03009). Les deux molécules sont en solution dans du DMSO en concentration finale respectivement à 3,5mM et 7mM.

Ces résultats confirment qu'un test basé sur l'activité HPPD quelle que soit l'origine de cette activité, permet d'identifier des activités HPPD présentant une tolérance à des inhibiteurs d'activité HPPD de famille des isoxazoles aussi bien que des tricétones.

### Exemple 2: Préparation et évaluation de l'activité d'HPPD chimères

L'objectif est de faire des fusions entre HPPD d'organismes phylogénétiquement distincts comme les plantes monocotylédones et dicotylédones ceci ayant plusieurs avantages:
- obtenir des HPPD ayant des caractéristiques en terme d'usage de codons plus favorables pour l'expression dans les hôtes pour lesquelles une tolérance aux inhibiteurs de l'HPPD est souhaitable mais difficile à obtenir avec une HPPD non chimérique,
- obtenir des HPPD ayant des caractéristiques en terme de faible affinité pour les inhibiteurs plus favorables,
- obtenir une activité HPPD in vitro ou in vivo avec un gène intéressant mais dont on ne dispose que d'un clone partiel.

On a testé des fusions de domaines N- et C-terminaux de plantes.

### 1) Construction

On réalise les fusions entre les régions N-terminales des dicotylédones, et la région C-terminal du maïs. On a permuté ces régions au sein des dicotylédones, proches phylogénétiquement.

La recherche de la zone de fusion s'est appuyée sur des comparaisons de séquences protéiques des différentes HPPD (figure 1). Par homologie avec l'HPPD de *Pseudomonas fluorescens*, on a identifié les parties N-terminales d'*Arabidopsis thaliana* et de *Daucus carota*, elles se terminent respectivement aux tyrosines 219 et 212. Le gène partiel de *Zea maïs* correspond donc à 80% de la partie C-terminale de la protéine.

On a choisi, comme zone d'échange, la région PINEP, très conservée chez les plantes. Ce qui est avant est la partie N-terminale et ce qui est après est la partie C-terminale. Par l'étude des séquences nucléotidiques, on a cherché à introduire un site de restriction, si possible unique, et qui ne modifie pas ou peu la séquence protéique, dans cette région. Le site de restriction AgeI qui répond à ces critères peut être obtenu en modifiant l'usage de codons pour les acides aminés E et P adjacents.

Le site AgeI a été introduit par mutagenèse dirigée en utilisant la méthode USE pour les HPPD d'*Arabidopsis thaliana* et de *Daucus carrota*, à l'aide des oligonucléotides dégénérés AgeAra, AgeCar et avec l'oligonucléotide de sélection USE AlwNI utilisable avec le vecteur pTRc.

| | |
|---|---|
| AgeAra | 5'pCCGATTAACGAACCGGTGCACGGAAC 3' |
| AgeCar | 5'pCCCTTGAATGAACCGGTGTATGGGACC 3' |
| USE AlwNI | 5'pCTAATCCTGTTACCGTTGGCTGCTGCC 3' |

On a utilisé la mutagenèse par PCR pour introduire, dans le même, temps le site SalI en fin de gène et le site AgeI chez *Zea maïs*, afin de faciliter le sous-clonage de toutes les fusions, dans le même vecteur, aux mêmes sites. Les amorces utilisées sont AgeMaïs et SalMaïsRev.

| | |
|---|---|
| AgeMaïs | 5' CCGCTCAACGAACCGGTGCACGGCACC 3' |
| SalMaïsRev | 5' GCAGTTGCTCGTCGACAAGCTCTGTCC 3' |

Après remplacement du fragment AgeI/SalI du clone d'HPPD d'*Arabidopsis thaliana* par le fragment AgeI/SalI de *Zea maïs* ou de *Daucus carrota*, on a obtenu les clones codant pour les HPPD chimèriques d'*Arabidopsis thaliana* / *Zea maïs* et d'*Arabidopsis thaliana* / *Daucus carrota* notés pFAM et pFAC (pour Fusion de N-terminal d'*Arabidopsis thaliana* avec un C-terminal de Maïs ou de Carotte). De la même façon, avec le clone d'HPPD de *Daucus carrota*, on a obtenu les clones pFCM et pFCA.

### 2) Activité in vivo

Le brunissement des colonies isolées, reflet de l'activité de l'HPPD, a été observé pour les fusions FAM, FAC, FCA et FCM. En comparaison avec les HPPD d'origine d'*Arabidopsis thaliana* (FAA) et de *Daucus carrota* (FCC), le brunissement a été noté sur une échelle de 1 à 10, 10 représentant le plus fort brunissement (comme décrit dans l'exemple 1)

| fragment N-terminal | fragment C-terminal | | |
|---|---|---|---|
| | ****C** | ****M** | ****A** |
| **FC*** | 5 | 2 | 2 |
| **FA*** | 9 | 9 | 10 |

Globalement, l'activité baisse pour chaque fusion en comparaison à l'HPPD fournissant le domaine N-terminal complet et début du domaine C-terminal.

Dans le cas ou le N-terminal provient d'*Arabidopsis thaliana*, la baisse d'activité est faible mais dans le cas où le N-terminal provient de *Daucus carrota*, l'activité mesurée chute plus nettement.

Ces expériences démontrent néanmoins que les HPPD chimères sont des enzymes actives, et qu'il est aujourd'hui tout à fait possible d'exprimer ces chimères dans les plantes, et par exemple d'exprimer une HPPD avec un N-terminal de dicotylédone et un C- terminale de monocotylédone dans les plantes.

## Revendications

1. Hydroxy-phényl pyruvate dioxygénase HPPD chimère fonctionnelle comprenant la partie N-terminale d'une première HPPD de plante associée à la partie C-terminale d'une deuxième HPPD d'origine phylogénétiquement différente, **caractérisée en ce que** lesdites parties N-terminale et C-terminale sont reliées par un peptide de liaison localisé entre 5 et 15 acides aminés en amont de l'acide aminé Asp161 par reference à la sequence de l'HPPD de *Pseudomonas* et **en ce que** l'HPPD chimère est différente de la première HPPD et de la deuxième HPPD.

2. HPPD chimère selon la revendication 1, **caractérisée en ce que** la partie C-terminale provient d'une HPPD de plante, de micro-organisme ou de champignon.

3. HPPD chimère selon l'une des revendications 1 ou 2, **caractérisée en ce que** la partie N-terminale de l'HPPD chimère provient d'une HPPD d'*Arabidopsis thaliana, Daucus carotta*, maïs ou blé.

4. HPPD chimère selon l'une des revendications 1 à 3, **caractérisée en ce que** la partie C-terminale de l'HPPD chimère provient d'une HPPD de plante choisie parmi les HPPD *d'Arabidopsis thaliana, Daucus carotta*, maïs ou blé.

5. HPPD chimère selon l'une des revendications 1 à 3, **caractérisée en ce que** la partie C-terminale de l'HPPD chimère provient d'une HPPD de bactérie, en particulier de *Pseudomonas,* plus particulièrement de *Pseudomonas fluorescens*.

6. Séquence d'acide nucléique codant pour une HPPD chimère selon l'une des revendications 1 à 5.

7. Gène chimère comprenant une séquence codante ainsi que des éléments de régulation en position 5' et 3' hétérologues pouvant fonctionner dans un organisme hôte, **caractérisé en ce que** la séquence codante comprend au moins une séquence d'acide nucléique codant pour une HPPD chimère selon la revendication 6.

8. Gène chimère selon la revendication 7, **caractérisé en ce que** l'organisme hôte est choisi parmi les bactéries, les levures, *Pichia*, les champignons, les baculovirus, les cellules végétales ou les plantes.

9. Gène chimère selon la revendication 7, **caractérisé en ce que** l'organisme hôte est une cellule végétale ou une plante.

10. Gène chimère selon la revendication 9, **caractérisé en ce qu'**il comprend en 5' de la séquence d'acide nucléique codant pour une HPPD chimère, une séquence d'acide nucléique codant pour un peptide de transit de plante, cette séquence étant disposée entre la région promotrice et la séquence codant pour l'HPPD chimère de manière à permettre l'expression d'une protéine de fusion peptide de transit/HPPD chimère.

11. Protéine de fusion peptide de transit/HPPD chimère, l'HPPD chimère étant définie selon l'une des revendications 1 à 5.

12. Vecteur de clonage et/ou d'expression pour la transformation d'un organisme hôte **caractérisé en ce qu'**il contient au moins un gène chimère selon l'une des revendications 7 à 10.

13. Procédé de transformation d'un organismes hôte, **caractérisé en ce que** l'on intègre de manière stable dans ledit organisme hôte au moins une séquence d'acide nucléique selon la revendication 6 ou un gène chimère selon l'une des revendications 7 à 10.

14. Procédé selon la revendication 13, **caractérisé en ce que** l'organisme hôte est une cellule végétale.

15. Procédé selon la revendication 14, **caractérisé en ce que** l'on régénère une plante à partir de la cellule végétale transformée.

16. Organisme hôte transformé, en particulier cellule végétale ou plante, **caractérisé en ce qu'**il contient une séquence d'acide nucléique selon la revendication 6 ou un gène chimère selon l'une des revendications 7 à 10.

17. Cellule végétale **caractérisé en ce qu'**elle contient une séquence d'acide nucléique selon la revendication 6 ou un gène chimère selon l'une des revendication 7 à 10.

18. Plante transformée, **caractérisée en ce qu'**elle contient des cellules transformées selon la revendication 17.

19. Plante selon la revendication 18, **caractérisée en ce qu'**elle est régénérée à partir des cellules transformées selon la revendication 17.

20. Plante transformée **caractérisée en ce qu'**elle est issue de la culture et/ou du croisement des plantes régénérées selon la revendication 19.

21. Plantes transformées selon l'une des revendications 18 à 20, **caractérisées en ce qu'**elles sont choisies parmi les monocotylédones, notamment les céréales, la canne à sucre, le riz et le maïs, ou les dicotyledones, notamment le tabac, la soja, le colza, le coton, la betterave et le trèfle.

22. Graines des plantes transformées selon l'une des revendications 18 à 21.

23. Procédé de contrôle des mauvaises herbes dans une surface d'un champ comprenant des graines selon la revendication 22 ou des plantes transformées selon l'une des revendications 18 à 21, lequel procédé consiste à appliquer dans la dite surface du champ une dose toxique pour les dites mauvaises herbes d'un herbicide inhibiteur d'HPPD, sans toutefois affecter de manière substantielle les graines ou plantes transformées avec le dit gène chimère selon l'invention.

24. Procédé de culture des plantes transformées selon l'une des revendications 18 à 21, lequel procédé consiste à planter les graines des dites plantes transformées selon la revendication 22 dans une surface d'un champ approprié pour la culture des dites plantes, à appliquer sur la dite surface du dit champ une dose toxique pour les mauvaises herbes d'un herbicide ayant pour cible l'HPPD en cas de présence de mauvaises herbes, sans affecter de manière substantielle les dites graines ou les dites plantes transformées, puis à récolter les plantes cultivées lorsqu'elles arrivent à la maturité souhaitée et éventuellement à séparer les graines des plantes récoltées.

25. Procédé selon l'une des revendications 23 ou 24, **caractérisé en ce que** l'inhibiteur d'HPPD est choisi parmi les isoxazoles, en particulier l'isoxaflutole, les dicétonitriles, en particulier la 2-cyano-3-cyclopropyl-1-(2-SO₂ CH₃-4-CF₃ phényl) propane-1,3-dione et la 2-cyano-3-cyclopropyl-1-(2-SO₂ CH₃-4-2,3 Cl₂ phényl) propane-1, 3-dione, les tricétones, en particulier la sulcotrione, ou les pyrazolinates.

## Claims

1. Functional chimeric hydroxyphenylpyruvate dioxygenase (HPPD) comprising the N-terminal part of a first plant HPPD combined with the C-terminal part of a second HPPD of phylogenetically different origin, **characterized in that** the said N-terminal and C-terminal parts are linked by a linking peptide located between 5 and 15 amino acids upstream of the amino acid Asp161, with reference to the *Pseudomonas* HPPD sequence, and **in that** the chimeric HPPD is different from the first HPPD and from the second HPPD.

2. Chimeric HPPD according to Claim 1, **characterized in that** the C-terminal part originates from a plant, microorganism or fungal HPPD.

3. Chimeric HPPD according to either of Claims 1 and 2, **characterized in that** the N-terminal part of the chimeric HPPD originates from an *Arabidopsis thaliana, Daucus carota*, maize or wheat HPPD.

4. Chimeric HPPD according to one of Claims 1 to 3, **characterized in that** the C-terminal part of the chimeric HPPD originates from a plant HPPD chosen from *Arabidopsis thaliana, Daucus carota*, maize or wheat HPPDs.

5. Chimeric HPPD according to one of Claims 1 to 3, **characterized in that** the C-terminal part of the chimeric HPPD originates from an HPPD of a bacterium, in particular of *Pseudomonas,* more particularly of *Pseudomonas fluorescens*.

6. Nucleic acid sequence which encodes a chimeric HPPD according to one of Claims 1 to 5.

7. Chimeric gene which comprises a coding sequence as well as heterologous regulatory elements, in the 5' and 3' positions, which are able to function in a host organism, **characterized in that** the coding sequence contains at least one nucleic acid sequence which encodes a chimeric HPPD according to Claim 6.

8. Chimeric gene according to Claim 7, **characterized in that** the host organism is selected from bacteria, yeasts, *Pichia*, fungi, baculoviruses, plant cells or plants.

9. Chimeric gene according to Claim 7, **characterized in that** the host organism is a plant cell or a plant.

10. Chimeric gene according to Claim 9, **characterized in that** it contains, in 5' of the nucleic acid sequence which encodes a chimeric HPPD, a nucleic acid sequence which encodes a plant transit peptide, with this sequence being arranged between the promoter region and the sequence encoding the chimeric HPPD so as to permit expression of a transit peptide/chimeric HPPD fusion protein.

11. Transit peptide/chimeric HPPD fusion protein, with the chimeric HPPD being defined in accordance with one of Claims 1 to 5.

12. Cloning and/or expression vector for transforming a host organism, **characterized in that** it contains at least one chimeric gene according to one of Claims 7 to 10.

13. Method for transforming a host organism, **characterized in that** at least one nucleic acid sequence according to Claim 6 or one chimeric gene according to one of Claims 7 to 10 is stably integrated into the said host organism.

14. Method according to Claim 13, **characterized in that** the host organism is a plant cell.

15. Method according to Claim 14, **characterized in that** a plant is regenerated from the transformed plant cell.

16. Transformed host organism, in particular a plant cell or plant, **characterized in that** it contains a nucleic acid sequence according to Claim 6 or a chimeric gene according to one of Claims 7 to 10.

17. Plant cell, **characterized in that** it contains a nucleic acid sequence according to Claim 6 or a chimeric gene according to one of Claim 7 to 10.

18. Transformed plant, **characterized in that** it contains transformed cells according to Claim 17.

19. Plant according to Claim 18, **characterized in that** it is regenerated from the transformed cells according to Claim 17.

20. Transformed plant, **characterized in that** it is derived by cultivating and/or crossing the regenerated plants according to Claim 19.

21. Transformed plants according to one of Claims 18 to 20, **characterized in that** they are selected from monocotyledones, in particular cereals, sugar cane, rice and maize, or dicotyledones, in particular tobacco, soya bean, rape, cotton, beetroot and clover.

22. Seeds from the transformed plants according to one of Claims 18 to 21.

23. Method for controlling weeds in an area of a field which contains transformed seeds according to Claim 22 or plants according to one of Claims 18 to 21, which method consists in applying, to the said area of the field, a dose of an HPPD inhibitor herbicide which is toxic for the said weeds, without, however, significantly affecting the seeds or plants which have been transformed with the said chimeric gene according to the invention.

24. Method for cultivating the plants which have been transformed according to one of Claims 18 to 21, which method consists in planting the seeds of the said transformed plants according to Claim 22 in an area of a field which is appropriate for cultivating the said plants, in applying, if weeds are present, a dose, which is toxic for the weeds, of a herbicide whose target is the HPPD to the said area of the said field, without significantly affecting the said transformed seeds or the said transformed plants, and in then harvesting the cultivated plants when they reach the desired stage of maturity and, where appropriate, in separating off the seeds of the harvested plants.

25. Method according to either of Claims 23 and 24, **characterized in that** the HPPD inhibitor is selected from isoxazoles, in particular isoxaflutole, diketonitriles, in particular 2-cyano-3-cyclopropyl-1-(2-SO₂CH₃-4-CF₃phenyl)-propane-1,3-dione and 2-cyano-3-cyclopropyl-1-(2-SO₂CH₃-4-2,3 Cl₂ phenyl)propane-1,3-dione, triketones, in particular sulcotrione, or pyrazolinates.

## Patentansprüche

1. Funktionsfähige chimäre Hydroxyphenylpyruvatdioxygenase, HPPD, umfassend den N-terminalen Abschnitt einer ersten planzlichen HPPD in Kombination mit dem C-terminalen Abschnitt einer zweiten HPPD mit phylogenetisch unterschiedlichem Ursprung, **dadurch gekennzeichnet, daß** die N-terminalen und C-terminalen Abschnitte durch ein Bindepeptid, das zwischen 5 und 15 Aminosäuren stromaufwärts der Aminosäure Asp161 in Bezug auf die Sequenz von HPPD *Pseudomonas* liegt, verbunden sind, und daß sich die chimäre HPPD von der ersten HPPD und von der zweiten HPPD unterscheidet.

2. Chimäre HPPD nach Anspruch 1**, dadurch gekennzeichnet, daß** der C-terminale Abschnitt von einer HPPD einer Planze, eines Mikroorganismus oder eines Pilzes stammt.

3. Chimäre HPPD nach einem der Ansprüche 1 oder 2, **dadurch gekennzeichnet, daß** der N-terminale Abschnitt der chimären HPPD aus einer HPPD aus *Arabidopsis thaliana, Daucus carota*, Mais oder Weizen stammt.

4. Chimäre HPPD nach einem der Ansprüche 1 bis 3, **dadurch gekennzeichnet, daß** der C-terminale Abschnitt der chimären HPPD aus einer pflanzlichen HPPD der Reihe HPPDs *Arabidopsis thaliana, Daucus carota*, Mais oder Weizen stammt.

5. Chimäre HPPD nach einem der Ansprüche 1 bis 3, **dadurch gekennzeichnet, daß** der C-terminale Abschnitt der chimären HPPD aus einer HPPD eines Bakteriums, insbesondere von *Pseudomonas,* ganz besonders von *Pseudomonas fluorescens,* stammt.

6. Nukleinsäuresequenz, die für eine chimäre HPPD nach einem der Ansprüche 1 bis 5 codiert.

7. Chimäres Gen, das eine Codiersequenz sowie heterologe Regulationselemente in 5'- und 3'-Position, die in einem Wirtsorganismus funktionsfähig sind, umfaßt, **dadurch gekennzeichnet, daß** die Codiersequenz mindestens eine Nukleinsäuresequenz, die für eine chimäre HPPD nach Anspruch 6 codiert, umfaßt.

8. Chimäres Gen nach Anspruch 7, **dadurch gekennzeichnet, daß** der Wirtsorganismus aus der Reihe der Bakterien, Hefen, *Pichia*, Pilze, Baculoviren, Pflanzenzellen oder Pflanzen stammt.

9. Chimäres Gen nach Anspruch 7, **dadurch gekennzeichnet, daß** es sich bei dem Wirtsorganismus um eine Pflanzenzelle oder eine Pflanze handelt.

10. Chimäres Gen nach Anspruch 9, **dadurch gekennzeichnet, daß** es in 5'-Position von der für eine chimäre HPPD codierenden Nukleinsäuresequenz eine Nukleinsäuresequenz, die für ein pflanzliches Transitpeptid codiert, umfaßt, wobei diese Sequenz zwischen der Promoterregion und der für die chimäre HPPD codierenden Sequenz liegt, sodaß die Expression eines Fusionsproteins Transitpeptid/chimäre HPPD ermöglicht wird.

11. Fusionspeptid Transitpeptid/chimäre HPPD, wobei die chimäre HPPD nach einem der Ansprüche 1 bis 5 definiert ist.

12. Klonierungs- und/oder Expressionsvektor für die Transformation eines Wirtsorganismus, **dadurch gekennzeichnet, daß** er mindestens ein chimäres Gen nach einem der Ansprüche 7 bis 10 enthält.

13. Verfahren zur Transformation eines Wirtsorganismus, **dadurch gekennzeichnet, daß** man mindestens eine Nukleinsäuresequenz nach Anspruch 6 oder ein chimäres Gen nach einem der Ansprüche 7 bis 10 stabil in den Wirtsorganismus integriert.

14. Verfahren nach Anspruch 13, **dadurch gekennzeichnet, daß** es sich bei dem Wirtsorganismus um eine Pflanzenzelle handelt.

15. Verfahren nach Anspruch 14, **dadurch gekennzeichnet, daß** man aus der transformierten Pflanzenzelle eine Pflanze regeneriert.

16. Transformierter Wirtsorganismus, insbesondere eine Pflanzenzelle oder Pflanze, **dadurch gekennzeichnet, daß** er/sie eine Nukleinsäuresequenz nach Anspruch 6 oder ein chimäres Gen nach einem der Ansprüche 7 bis 10 enthält.

17. Pflanzenzelle, **dadurch gekennzeichnet, daß** sie eine Nukleinsäuresequenz nach Anspruch 6 oder ein chimäres Gen nach einem der Ansprüche 7 bis 10 enthält.

18. Transformierte Pflanze, **dadurch gekennzeichnet, daß** sie transformierte Zellen nach Anspruch 17 enthält.

19. Pflanze nach Anspruch 24, **dadurch gekennzeichnet, daß** sie aus transformierten Zellen nach Anspruch 17 regeneriert wurde.

20. Transformierte Pflanze, **dadurch gekennzeichnet, daß** sie durch Kultur und/oder Kreuzung von regenerierten Pflanzen nach Anspruch 19 entstanden ist.

21. Transformierte Pflanzen nach einem der Ansprüche 18 bis 20, **dadurch gekennzeichnet, daß** sie aus der Reihe der einkeimblättrigen Pflanzen, insbesondere Getreide, Zuckerrohr, Reis und Mais, oder der zweikeimblättrigen Pflanzen, insbesondere Tabak, Sojabohne, Raps, Baumwolle, Zucker/Futterrübe und Klee stammen.

22. Samen von transformierten Pflanzen nach einem der Ansprüche 18 bis 21.

23. Verfahren zur Bekämpfung von Unkräutern in einer Oberfläche eines Felds, das Samen nach Anspruch 22 oder transformierte Pflanzen nach einem der Ansprüche 18 bis 21 umfaßt, das darin besteht, daß man in die Oberfläche des Felds eine für diese Unkräuter toxische Dosis eines HPPD-Hemmer-Herbizids ausbringt, ohne jedoch die Samen oder Pflanzen, die mit dem erfindungsgemäßen chimären Gen transformiert wurden, wesentlich zu schädigen.

24. Verfahren zum Kultivieren von transformierten Pflanzen nach einem der Ansprüche 18 bis 21, das darin besteht, daß man die Samen dieser transformierten Pflanzen nach Anspruch 22 in die Oberfläche eines für die Kultur dieser Pflanzen geeigneten Felds sät, falls Unkräuter vorhanden sind in die Oberfläche des Felds eine für diese Unkräuter toxische Dosis eines HPPD-Hemmer-Herbizids ausbringt, ohne die Samen oder Pflanzen, die transformiert wurden, wesentlich zu schädigen und anschließend die kultivierten Pflanzen bei Erreichen der gewünschten Reife erntet und gegebenenfalls die Samen von den geernteten Pflanzen abtrennt.

25. Verfahren nach einem der Ansprüche 23 oder 24, **dadurch gekennzeichnet, daß** der HPPD-Hemmer aus der Reihe der Isoxazole, insbesondere Isoxaflutol, der Diketonitrile, insbesondere 2-Cyano-3-cyclopropyl-1-(2-SO₂CH₃-4-CF₃-phenyl)propan-1,3-dion und 2-Cyano-3-cyclopropyl-1-(2-SO₂CH₃-4-2,3-Cl₂-phenyl)propan-1,3-dion, der Triketone, insbesondere Sulcotrion, oder der Pyrazolinate stammt.


### LISTE DE SEQUENCES

(1) INFORMATIONS GENERALES:
   (i) DEPOSANT:
      (A) NOM: RHONE-POULENC AGROCHIMIE
      (B) RUE: 14-20 Rue Pierre BAIZET
      (C) VILLE: LYON
      (E) PAYS: France
      (F) CODE POSTAL: 69009
   (ii) TITRE DE L'INVENTION: Hydroxy-phényl pyruvate dioxygénase chimère, séquence d'ADN et obtention de plantes contenant un tel gène, tolérantes aux herbicides
   (iii) NOMBRE DE SEQUENCES: 5
   (iv) FORME DECHIFFRABLE PAR ORDINATEUR:
      (A) TYPE DE SUPPORT: Floppy disk
      (B) ORDINATEUR: IBM PC compatible
      (C) SYSTEME D'EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 26 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 1:
      CCGATTAACG AACCGGTGCA CGGAAC 26
(2) INFORMATIONS POUR LA SEQ ID NO: 2:
   (i) CARACTERISTIQUES DE LA SEQUENCE:
      (A) LONGUEUR: 27 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 2:
      CCCTTGAATG AACCGGTGTA TGGGACC 27
(2) INFORMATIONS POUR LA SEQ ID NO: 3:
   (i) CARACTERISTIQUES DE LA SEQUENCE:
      (A) LONGUEUR: 27 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 3:
      CTAATCCTGT TACCGTTGGC TGCTGCC 27
(2) INFORMATIONS POUR LA SEQ ID NO: 4:
   (i) CARACTERISTIQUES DE LA SEQUENCE:
      (A) LONGUEUR: 27 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 4:
      CCGCTCAACG AACCGGTGCA CGGCACC 27
(2) INFORMATIONS POUR LA SEQ ID NO: 5:
   (i) CARACTERISTIQUES DE LA SEQUENCE:
      (A) LONGUEUR: 27 paires de bases
      (B) TYPE: nucléotide
      (C) NOMBRE DE BRINS: simple
      (D) CONFIGURATION: linéaire
   (ii) TYPE DE MOLECULE: ADN
   (xi) DESCRIPTION DE LA SEQUENCE: SEQ ID NO: 5:
      GCAGTTGCTC GTCGACAAGC TCTGTCC 27